# EUROPEAN PATENT APPLICATION

(11) **EP 4 582 548 A1**
(43) Date of publication of application: **09.07.2025**
(21) Application number: 24203932.9
(22) Date of filing: 01.10.2024
(51) Int. Cl.: C12P 5/02, C02F 11/04, C12M 1/00, C12M 1/107

(54) **PROCESS FOR THE CONTINUOUS PRODUCTION OF BIOMETHANE FROM BIOMASS WITH CO2 SEQUESTRATION**

(30) Priority: 04.01.2024 IT 202400000108
(71) Applicant: Ingenia S.r.l., 60019 Senigallia AN (IT)
(72) Inventor: Rossi, Tommaso, 60019 Senigallia AN (IT); Marini, Filippo, 60019 Senigallia AN (IT)
(74) Representative: Perani & Partners S.p.A.

(57) **Abstract**

Process for the continuous production of biomethane from biomasses, comprising the following steps:
i) preparing a biomass (1);
ii) diluting and alkalizing the biomass (1) by adding a basic mixture from algal culture and an alkaline solution (2), up to a pH comprised between 9.0 and 13.0;
iii) subjecting the diluted and alkalized biomass to alkaline hydrolysis, at a temperature comprised between 55 and 90°C;
iv) subjecting the hydrolysed biomass to ammonia stripping by diffusion of a biogas, to obtain a hydrolysed and deammonified biomass;
v) neutralizing the hydrolysed and deammonified biomass, coming from step iv), by diffusing the CO₂ present in a biogas, to obtain a neutralized biomass;
vi) subjecting the neutralized biomass, coming from step v), to anaerobic digestion, to obtain a biogas (4) and a digestate (5), and recycling said biogas (4) in step v), and subsequently from step v) in step iv);
vii) subjecting the digestate (5) to a separation step, to obtain a solid or semi-solid digestate (6) and a clarified digestate (7);
viii) feeding the clarified digestate (7) into a prepared algal culture to obtain a basic mixture (8) from the algal culture;
ix) recycling said basic mixture (8) from algal culture according to one of the following alternatives:
ix.i) the basic mixture (8) of algal culture is recycled in step ii) of dilution and alkalization of the biomass (1), if said basic mixture of algal culture (8) has a pH ≥ 9.0; or
ix.ii) the basic mixture (8) of algal culture is recycled upstream of step viii), if said basic mixture of algal culture (8) has a pH < 9.0;

x) subjecting the biogas, coming from step vi), to purification, by removing gaseous ammonia and residual moisture, to obtain biomethane.

## Description

### FIELD OF THE INVENTION

The present invention relates to a process for the continuous production of biomethane from biomass, by anaerobic digestion.

### STATE OF THE ART

It is known in the state of the art, for the production of biomethane from biomass, to use anaerobic digestion (or stabilization), a biological process that allows the degradation of the organic substance by particular anaerobic microorganisms. These microorganisms are able to survive and reproduce in anoxic environments, that is, in the absence of oxygen and are able to carry out methanogenesis, producing a combustible gas called biogas composed mainly of methane, CO₂ and H₂S.

Biomethane is considered one of the fuels of greatest interest, both for its ease of use in the transport sector and for its potential for dissemination on a geographic scale. As known in the state of the art, biomethane is obtained by the purification (or "upgrading") of biogas obtained from the anaerobic fermentation of organic matrices.

Biogas is, in fact, the final product of the anaerobic digestion of organic biomass, and consists of a mixture composed mainly of methane (CH₄), carbon dioxide (CO₂) and hydrogen sulphide (H₂S).

Consequently, the purification (or "upgrading") of biogas into biomethane consists essentially in the removal of carbon dioxide (CO₂) and hydrogen sulphide (H₂S), so as to obtain a gas composed mainly of methane, therefore suitable for being introduced into the transport and distribution networks, according to the criteria defined by the UNI/TR 11537 "Input of biomethane into the natural gas transport and distribution networks", and subsequent amendments, the main reference legislation. WO2017/216720 describes a process for the production of biomethane from biomass, characterized by a step of dilution, alkalization and hydrolysis of a biomass and the subsequent neutralization by reduction of the CO₂ present in a biogas obtained by anaerobic digestion, thus allowing the purification of said biogas to biomethane.

### Problems of the background art

The methods known to the state of the art, which provide for an anaerobic digestion step, generally present difficulties in the management of biomasses particularly loaded with nitrogen, such as pollen, agricultural waste, Organic Fraction of Urban Solid Waste (FORSU), and/or civil and/or industrial sludge. In fact, the nitrogen present in the biomass used as a raw material tends to accumulate in the anaerobic digester in the form of ammoniacal nitrogen. The latter is a component capable of interfering with the action carried out by methanogenic bacteria and, beyond a certain concentration, of completely inhibiting anaerobic fermentation, as well as determining the possible formation of an effluent digestate rich in ammoniacal nitrogen, which can only be used as an agricultural fertilizer or as an additive for the production of compost. This leads to an increase in difficulties and operating costs related to the spreading of digestate in the agricultural sector or for the production of compost, as well as energy costs for the development of the related facilities.

In addition, the methods known to the state of the art, which provide for upgrading from biogas to biomethane, by separating CO₂, present difficulties in managing the residual gaseous CO₂ from the separation, which is normally emitted into the atmosphere (Off Gas) without being sequestered, resulting in the emission of a greenhouse gas, with difficulties in management due to the impurities present. Alternatively, CO₂ is generally sequestered for use in the food field, with considerable consequent costs, in terms of energy self-consumption and plant management for food purification, liquefaction and subsequent transport.

The process described in WO2017/216720 provides for a significant consumption of reagent used in the alkalization step to raise the pH; moreover, the residual digestate, being rich in bicarbonate ion and therefore being difficult to use for agronomic spreading, must be mainly disposed of in the sewer, with the need for purification to reach a concentration of ammoniacal nitrogen and other pollutants such as to be suitable for discharge into the sewer or other receiver, with certain energy and plant costs.

Furthermore, in this process, the consumption of reagent is directly proportional to the amount of CO₂ present in the biogas and, consequently, this consumption is associated with an energy and economic cost of managing the plant for the implementation of the process, especially on an industrial scale.

Finally, in accordance with current legislation, in particular UNI/TS 11567, the reagent consumption of the process in question is part of the calculation of the greenhouse gas, or GHG ("Greenhouse Gases") budget, negatively affecting the overall environmental performance, in terms of savings on greenhouse gas emissions.

### SUMMARY OF THE INVENTION

In this context, the technical task underlying the present invention is to provide a process for the continuous production of biomethane from biomass, comprising the following steps:
i) preparing a biomass 1;
ii) diluting and alkalizing the biomass 1 by adding a basic mixture from algal culture and an alkaline solution 2, up to a pH comprised between 9.0 and 13.0;
iii) subj ecting the diluted and alkalized biomass to alkaline hydrolysis, at a temperature comprised between 55 and 90°C;
iv) subjecting the hydrolysed biomass to ammonia stripping by diffusion of a biogas, to obtain a hydrolysed and deammonified biomass;
v) neutralizing the hydrolysed and deammonified biomass, by diffusing the CO₂ present in a biogas, to obtain a neutralized biomass;
vi) subjecting the neutralized biomass, coming from step v), to anaerobic digestion, to obtain a biogas 4 and a digestate 5, and recycling said biogas 4 in step v), and subsequently from step v) in step iv);
vii) subjecting the digestate 5 to a separation step, to obtain a solid or semi-solid digestate 6 and a clarified digestate 7;
viii) feeding the clarified digestate 7 into a prepared algal culture to obtain a basic mixture 8 from the algal culture;
ix) recycling said basic mixture 8 according to one of the following alternatives:
   ix.i) the basic mixture 8 is recycled in step ii) of dilution and alkalization of the biomass 1, if said basic mixture 8 has a pH ≥ 9.0; or
   ix.ii) the basic mixture 8 is recycled upstream of step viii), if said basic mixture 8 has a pH < 9.0;
x) subjecting the biogas, coming from step vi), to purification, by removing gaseous ammonia and residual moisture, to obtain biomethane.

### Advantages of the invention

The process of the invention allows the continuous production of biomethane from biomass.

In particular, compared to the process described in WO2017/216720, the process of the invention is more environmentally sustainable, allowing:
- exploiting the digestate, in particular the clarified digestate, obtained from the anaerobic digestion of biomass to feed an algal culture;
- exploiting the bicarbonate that is produced in the neutralization step of hydrolysed and alkalized biomass as nourishment for the growth of algae in algal culture;
- minimizing the need for reagent used to carry out the alkalization step. The process of the invention allows, in fact, by means of said algal culture, producing a basic mixture that is recycled as a reagent in the biomass alkalization step;
- obtaining a more favourable overall GHG greenhouse gas balance.

In addition, compared to the process described in WO2017/216720, the process of the invention, providing an ammonia stripping step, allows the ammoniacal nitrogen content in the neutralized biomass to be minimized, before it is subjected to anaerobic digestion. Accordingly, the process of the invention advantageously allows a greater variety of biomasses, including those particularly rich in nitrogen, to be employed as feedstock, while improving the qualitative and/or quantitative production efficiency of the biomethane.

Furthermore, the ammonia stripping step allows an anaerobic digested biomass with ammoniacal nitrogen content within a certain desired range of values to be obtained, in such a way as to make the liquid digestate downstream of the anaerobic digestion step suitable and optimal to be used to feed a predisposed algal culture.

### DESCRIPTION OF THE FIGURES

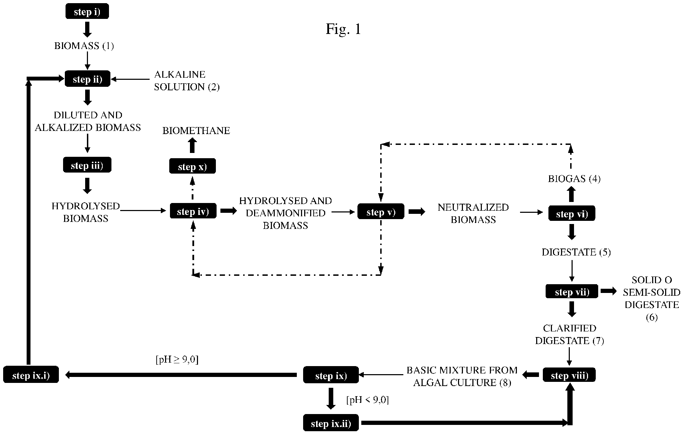

Figure 1: flow chart of the process according to the present invention, in which the steps and products obtained by said process are shown. Note that biogas recycling is shown by dashed lines/arrows.

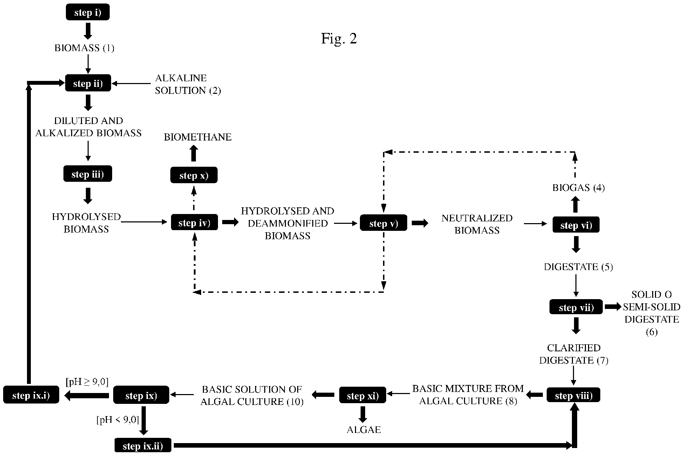

Figure 2: flow chart of a preferred embodiment of the process according to the present invention, in which the steps and products obtained by said process are shown. In particular, it is noted that this preferred embodiment provides for a further step xi) of separating the basic mixture 8, coming from step viii), to obtain algae and a basic solution 10 from algal culture. Note that biogas recycling is shown by dashed lines/arrows.

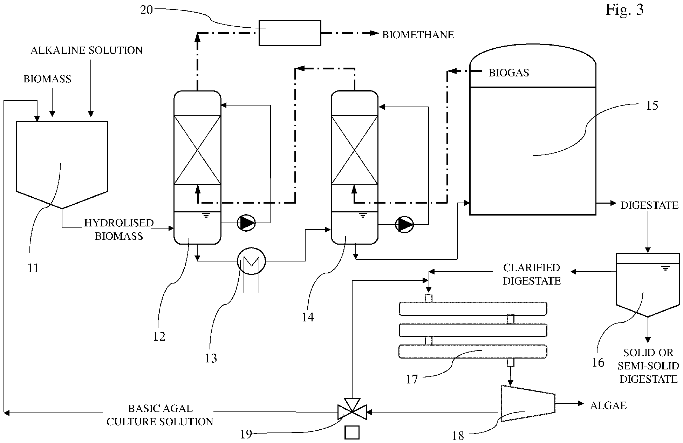

Figure 3: schematic representation of an embodiment of the system for the implementation of the method according to the present invention, comprising a reactor 11, a drip or spray column reactor 12, a heat exchanger 13, a column reactor 14, a digester 15, an algal culture 17, a separator 18, a valve 19, a reactor 20. The column reactor 12 is equipped with a lower accumulator with a recirculation system, configured to recycle the hydrolysed biomass to be subjected to stripping of the ammonia at the head of the reactor column 12. The column reactor 14 is also equipped with a lower accumulator with a recirculation system, configured to recirculate the biomass to be neutralized at the head of the reactor column 14. Note that biogas recycling is shown by dashed lines/arrows.

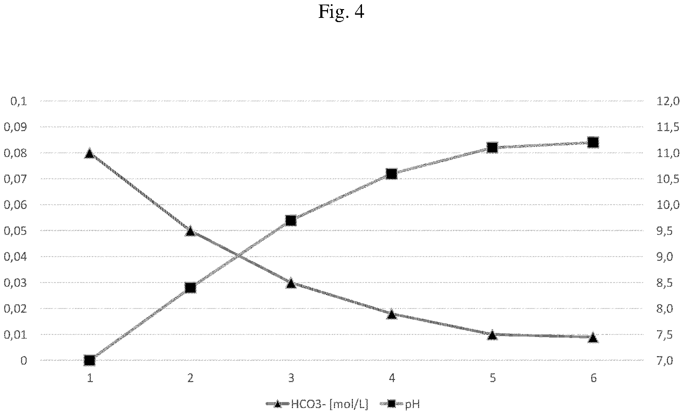

Figure 4: Graph in which the trend of the pH (curve with square indicator) and the concentration of bicarbonates (curve with triangular indicator) during the algal cultivation step of an embodiment of the process according to the present invention are shown.

### DETAILED DESCRIPTION OF THE INVENTION

### Definitions

Biomass means any material having a biodegradable fraction of biological origin, coming from forestry and/or agriculture, including plant and/or animal substances; any product, waste and residue of the wood and paper processing industry; and/or all products, waste and organic residues having a biodegradable fraction, derived from the biological activity of animals and humans, such as those contained in industrial and urban waste.

'Organic sludge' means sludge produced from the water line of wastewater purification plants of urban and/or industrial origin, preferably characterized by a high moisture content, preferably a moisture content between 95 and 99%.

Organic scraps means the wet waste of municipal solid waste, consisting of food scraps and other waste, having a biodegradable fraction, and/or agro-industrial by-products, such as those derived from meat processing, olive pressing, processing of agricultural products, and/or winemaking.

Treated or untreated residual sludge coming from urban, domestic, agricultural, and/or industrial wastewater treatment plants means sludge produced by urban wastewater and/or domestic water purification processes; sludge coming from septic tanks; and/or sludge coming from agro-industrial wastewater treatment plants.

Treated or untreated residual sludges coming from production processes are: sludges of agri-food origin, from the paper and wood industry, preferably sludges whose organic products and/or by-products are derived from composting plants, agricultural and forestry activities, livestock, horticulture and food preparation; sludges derived from civil processing; sludges coming from industrial wastewater treatment plants; sludges coming from the textile and leather industries; and/or sludges coming from the petroleum industry or derived from organic and inorganic chemical processes.

By basic algal culture mixture is meant the liquid product that is obtained from an algal culture, which is essentially a cultivation broth (or culture) comprising algae suspended in a basic liquid solution.

Deammonified biomass means a biomass treated by ammonia stripping (or degassing) processes, having an ammoniacal nitrogen content between 30 and 850 mg/L, preferably between 300 and 650 mg/L, preferably between 320 and 450 mg/L, preferably equal to 350 mg/L.

Neutralized biomass means a biomass having a pH value between 6 and 8, preferably 7.0 or preferably 7.5 to 7.7.

Chemical Oxygen Demand (COD) means the amount of oxygen (expressed in mg O₂/l) necessary for the complete oxidation of organic and inorganic compounds present in a liquid sample.

Algal culture refers to a cultivation of algae, carried out in dedicated circular nurseries ("raceway ponds") in the open air, or prepared in dedicated photobioreactors (PBRs).

Algae means unicellular or multicellular autotrophic plant organisms, including macroalgae, microalgae and/or cyanobacteria, capable of using dissolved carbon dioxide and bicarbonates as a carbon source for their own metabolism and growth, and releasing hydroxyl ion, alkalizing (or raising the pH) the algal culture liquid. Preferably, the algae are able to live and proliferate in basic environments, preferably at pH values between 7 and 13.

Solid or semi-solid digestate refers to the concentrated residue obtained by the static or dynamic separation of the digestate. Preferably, the solid digestate has a dry matter concentration of from 20 to 90% (w/w), while the semi-solid digestate has a dry matter concentration of from 6 to 20% by weight (w/w).

By clarified digestate is meant the clarified liquid that is obtained following the static or dynamic separation of the digestate.

Biomethane refers to the gas, mainly composed of methane, obtained from the purification of biogas.

### A process for the production of biomethane from biomass

As mentioned above, an object of the present invention is a process for the continuous production of biomethane from biomass, comprising the steps described below.

### i) Preparing a biomass

In a step i) of the process according to the present invention a biomass 1 is prepared.

Preferably, the biomass 1 is chosen from: biological sludge, algae, organic waste or by-products, organic scraps, preferably organic scraps coming from separate and undifferentiated collection, process waters, treated and untreated residual sludge, preferably coming from urban, domestic and/or industrial wastewater treatment plants, and/or treated and untreated residual sludge coming from production processes.

Advantageously, the process of the invention enables the treatment of both wet biomasses and dry biomasses (or with low moisture), wherein: wet biomasses are understood to mean biomasses having a moisture content of between 70 and 99% by weight; dry biomasses are understood to mean spadable biomasses having a moisture content of less than 70% by weight.

### ii) Diluting and alkalizing biomass

In a step ii) of the process according to the present invention the biomass 1 is subjected to dilution and alkalization, by the addition of a basic mixture from algal culture and of an alkaline solution 2, to a pH comprised between 9.0 and 13.0, preferably comprised between 11.0 and 13.0, preferably equal to 11.6.

It is noted that the basic algal culture mixture, in step ii) of the process, performs the function of diluent and, at the same time, of alkalizing agent. Consequently, by contributing to raising the pH in step ii), it advantageously enables reduction in the requirement for alkaline solution 2 used for alkalization and, in particular, for the adjustment of the pH within the desired range of values.

Preferably, the alkaline solution 2 is an aqueous solution comprising a strong base, preferably an alkaline or alkaline-earth metal hydroxide, preferably sodium hydroxide, potassium hydroxide, or mixtures thereof, preferably at 20% by weight on the total volume of the alkaline solution 2 (w/v).

Preferably, in step ii) the biomass 1 is subjected to dilution and alkalization, up to a content of volatile solids comprised between 0.5 and 10.0%, preferably comprised between 0.7 and 8.0%, preferably comprised between 0.8 and 1.5, preferably equal to 1.0% by weight with respect to the volume of said biomass 1 (w/v).

Advantageously, the dilution and alkalization step (ii) allows for greater solubilization of the solids present in the biomass through the subsequent hydrolysis step, a solubilization that increases the digestibility of the input material in the subsequent anaerobic digestion step and, thus, the overall energy yield of the process.

### iii) Subjecting the diluted and alkalized biomass to alkaline hydrolysis

In a step iii) of the process in accordance with the present invention, the diluted and alkalized biomass, coming from step ii), is subjected to alkaline hydrolysis, at a temperature between 55 and 90°C, preferably equal to 80°C.

Advantageously, step iii) of alkaline hydrolysis allows, with an energy consumption between 0.95 and 2.85 kWh per cubic meter of biomass (kWh/m³), the production of a hydrolysed biomass, characterized by an efficiency in the solubilization the solids present in said biomass greater than 90%.

The solubilization efficiency of solids present in biomass is measured in terms of the ratio between the chemical oxygen demand ("Chemical Oxygen Demand" or COD), determined on the clear fraction (supernatant) obtained by filtration and centrifugation of the hydrolysed material with respect to the total COD of the hydrolysed material.

Still advantageously, the increase in the solubilization of the solids present in the biomass allows, in turn, an increase in the digestibility of the material in the subsequent steps, in particular in the anaerobic digestion step, ensuring an overall energy increase of the process.

The alkaline hydrolysis step iii) is carried out for a time comprised between 30 and 120 minutes, preferably equal to 90 minutes.

Preferably, the hydrolysed biomass, coming from step iv), has a pH comprised between 9.0 and 13.0.

Note that preferably steps ii) and iii) are conducted in a dedicated reactor 11, preferably a CSTR ("continuous stirred tank reactor") chemical reactor.

It should be noted that the hydrolysed biomass, coming from step iii), is in alkaline conditions, following step ii); the nitrogen coming from biomass 1 is, in fact, mostly found in the hydrolysed biomass in the form of dissolved gaseous ammonia, which is therefore suitable to be subjected to stripping of ammonia in the subsequent step of the process in accordance with the present invention.

### iv) Subjecting the hydrolysed biomass to ammonia stripping

In a step iv) of the process according to the present invention the hydrolysed biomass, coming from step iii), is subjected to stripping (or degassing) of the ammonia, by means of the diffusion of a biogas, to obtain a hydrolysed and deammonified biomass.

According to a preferred embodiment, the ammonia stripping step (iv) is conducted at a temperature between 55 and 90°C, preferably 80°C.

Still preferably, the ammonia stripping step iv) is carried out at a temperature equal to or lower than, preferably equal to, the conduction temperature of step iii) of the process of the invention. This advantageously allows step iv) to be carried out without the need to heat the system further, improving the overall energy efficiency of the process.

Preferably, the process step iv) is carried out in a drip column reactor 12, loaded with filler material, or in a spray or Venturi scrubber type reactor 12, said reactor 12 being equipped with a lower storage tank (or accumulator) with a recirculation system, configured to recirculate the hydrolysed biomass subjected to ammonia stripping at the head of the reactor 12 column.

Advantageously, this recirculation system for the hydrolysed biomass subjected to ammonia stripping allows the ammonia stripping efficiency to be regulated. This advantageously allows adjustment of the concentration of ammoniacal nitrogen in the treated biomass within the desired range of values, so as to obtain a deammonified biomass, suitable to be used in the subsequent feeding step of an algal culture of the process according to the present invention.

Preferably, the filler material is pellets or spheres, Cascade Mini-Rings (CMR), IMTP Rings, Nutter rings, Raschig rings, Lessing rings, Pall rings, Bialecki rings, preferably said filler material being plastic or metal material.

Still preferably, the filler material has a porosity greater than 0.5, preferably comprised between 0.8 and 0.98, and preferably a specific surface comprised between 60 and 500 m²/m³.

Preferably, the hydrolysed and deammonified biomass is cooled, preferably to a temperature of 40°C.

It should be noted that the cooling of the hydrolysed and deammonified biomass is preferably conducted in a heat exchanger 13, configured for thermal recovery, advantageously allowing the energy consumption of the process to be minimized.

### v) Neutralizing hydrolysed and deammonified biomass

In a step v) of the process according to the present invention, the hydrolysed and deammonified biomass coming from step iv) is subjected to neutralization by diffusion of the CO₂ present in a biogas to obtain neutralized biomass.

It should be noted that the diffusion of the CO₂ present in the biogas allows the solubilization of the CO₂ itself and its conversion into carbonic acid in solution in the hydrolysed and deammonified biomass. The acid-base neutralization reaction therefore takes place between the hydrolysed and deammonified biomass, which is in alkaline conditions, as a result of step ii), and the H⁺ ions produced by the dissociation of carbonic acid in solution. In turn, the dissociation of the carbonic acid in solution enriches the neutralized biomass in bicarbonates, advantageously making this biomass suitable to be used in the subsequent steps of the process, in particular to feed a predisposed algal culture.

It should also be noted that, at the same time, the neutralization step v) advantageously also allows the concentration of H₂S present in the biogas to be reduced, which, due to its acidic nature, also reacts with the hydrolysed and deammonified biomass, which is in alkaline conditions.

Preferably, the diffusion of the CO₂ in the neutralization step v) is carried out in a drip column reactor 14, loaded with filler material, and saturated with a biogas.

Preferably, the filler material is pellets or spheres, Cascade Mini-Rings (CMR), IMTP Rings, Nutter rings, Raschig rings, Lessing rings, Pall rings, Bialecki rings, preferably said filler material being plastic or metal material.

Preferably, the hydrolysed and deammonified biomass feeds the column reactor 14 from the top down, while the biogas flow is fed from the bottom up.

Preferably, the filler material of the reactor 14 has a porosity greater than 0.5, preferably comprised between 0.8 and 0.98 and preferably a specific surface comprised between 60 and 500 m²/m³.

It should be noted that said column reactor 14 is preferably provided with a dedicated lower storage tank (or accumulator) with recirculation system, configured to recirculate the biomass to be neutralized at the head of the reactor column 14.

Advantageously, such a recirculation system of the biomass to be neutralized allows the efficiency of the neutralization process to be adjusted, so as to obtain a pH value of the neutralized biomass within the desired range of values, i.e. comprised between 6 and 8, preferably equal to 7.0 or preferably comprised between 7.5 and 7.7, so as to be able to be suitably used in the subsequent steps of the process.

Preferably, the biogas is kept in the column reactor 14 at a pressure between 0.01 and 0.1 bar, preferably 0.05 bar.

Still preferably, the hydrolysed and deammonified biomass feeds the column reactor 14 from above and is preferably recirculated with downward flow, in gravity from top to bottom, until it is completely neutralized.

### vi) Subjecting neutralized biomass to anaerobic digestion

In a step vi) of the process according to the present invention subjecting the neutralized biomass, coming from step v), to anaerobic digestion, to obtain a biogas 4 and a digestate 5, and recycling said biogas 4 in step v), and subsequently from step v) in step iv).

It should be noted that, in accordance with step vi), the biogas 4 is recycled in step v), in which it is used to carry out the neutralization by diffusion of CO₂; consequently, from step v) a biogas purified from CO₂ is obtained, which is recycled in step iv) and then used to carry out the stripping of ammonia.

Advantageously, thanks to the high digestibility of the neutralized biomass coming from step v), the yield of biogas production 4 from step vi) of anaerobic digestion is particularly high, preferably with an average residence time between 1 and 20 days, preferably equal to 2 days or equal to 15 days and with a high energy yield.

Preferably, the anaerobic digestion in step vi) is carried out in a dedicated digester 15, equipped with a biogas recirculation system, configured to feed the biogas produced by the anaerobic digestion into the reactor 14, wherein said reactor 14 is, in turn, configured to feed the biogas exiting into the reactor 12.

### vii) Subjecting the digestate to separation

In a step vii) of the process according to the present invention, the digestate 5 is subjected to a separation, to obtain a solid or semi-solid digestate 6 and a clarified digestate 7.

Note that the step vii) of separating the digestate 5 is preferably carried out by static or dynamic thickening, or by mechanical separation, preferably by centrifugation, microfiltration or filter pressing.

Still preferably, the step vii) of separating the digestate 5 is carried out in a dedicated separator 16, consisting of a static gravity thickener or a dynamic separator for centrifugation, microfiltration or filter pressing.

It should be noted that steps iv) and v) respectively allow regulation of the concentration of ammoniacal nitrogen in solution within a specific range of values and to enrich the solution in bicarbonates, which are produced by the dissociation in solution of carbonic acid that is formed by diffusion of CO₂; consequently, the clarified digestate 7 has a concentration of ammoniacal nitrogen and bicarbonates such as to be suitable to be used as a medium for feeding an algal culture.

### viii) Feeding the clarified digestate in a prepared algal culture

In a step viii) of the process according to the present invention, clarified digestate 7 is fed into a prepared algal culture 17 to obtain a basic mixture 8 from the algal culture.

Preferably, said algal culture 17 comprises algae, preferably microalgae or cyanobacteria, preferably of the species Spirulina, Chlorella, Klamath, Neochloris, Asparagopsis.

It should be noted that, during step viii), the algae feed on the bicarbonate ions and ammoniacal nitrogen present in the solution, for their own growth, producing hydroxyl ions in the culture water and forming the basic mixture 8 from algal culture.

### ix) Recycling the basic mixture

In a step ix) of the process according to the present invention the basic mixture 8 is recycled from algal culture according to one of the following alternatives:
ix.i) the basic mixture 8 is recycled in step ii) of dilution and alkalization of biomass 1, if said basic mixture 8 has a pH ≥ 9.0, preferably between 9.0 and 13.0, preferably between 10.0 and 12.0, preferably equal to 11.2; or
ix.ii) the basic mixture 8 is recycled upstream of step viii), if said basic mixture 8 has a pH < 9.0.

It should be noted that, according to alternative ix.i), the basic mixture 8 of algal culture is recycled in step ii); consequently, since the basic mixture 8 of algal culture comprises algae in suspension in basic solution, this alternative advantageously allows the reuse of the basic solution of algal culture and, at the same time, of the algae, which are recycled in step ii) as biomass 1.

Also advantageously, recycling the basic mixture 8 in step ii) reduces the need for the reagent, i.e. alkaline solution 2, required to carry out this step.

Still advantageously, alternative ix.ii) allows the basic mixture 8 to be re-introduced in step viii), thus allowing it to be reused as a means of feeding the algal culture in addition to the clarified digestate 7.

Preferably, the recycling of the basic mixture 8 in accordance with the aforementioned alternative steps ix.i) and ix.ii) is carried out by means of a dedicated flow diverter valve 19, configured to direct the flow of the basic mixture 8 out of the algal culture respectively:
- to the input of the reactor 11; or
- to the input of the the algal culture 17.

### x) Purifying the biogas coming from step vi)

In a step x) of the process according to the present invention, the biogas coming from step vi) is subjected to purification, by removing gaseous ammonia and residual moisture, to obtain biomethane.

Advantageously, the process according to the present invention makes it possible to obtain biomethane with a residual molar CO₂ concentration of less than 2.5%, preferably less than 0.2%.

It is noted that the biogas coming from step vi) is biogas 4, produced by anaerobic digestion, after being recycled sequentially in steps v) and iv).

Preferably, the removal of the gaseous ammonia in step x) is carried out by acid scrubbing, preferably by washing the biogas stream with a sulfuric acid solution, preferably a 20 wt% aqueous sulfuric acid solution based on the total weight of the solution (w/w). This allows the reduction of gaseous ammonia and its recovery in the form of ammonium sulfate.

Preferably, the reduction of the residual moisture in step x) is carried out by condensation.

Preferably, the removal of the gaseous ammonia in step x) is carried out in a dedicated columnar reactor 20, loaded with filler material, or spray or Venturi scrubber, in which the flow of biogas to be purified is preferably circulated from the bottom upwards, while the sulfuric acid solution is percolated counter-currently from the top downwards.

Preferably, the reactor filler material 20 has a porosity greater than 0.2, preferably comprised between 0.5 and 0.9, and preferably has a specific surface area comprised between 20 and 600 m²/m³.

Still preferably, the removal of the residual moisture in step x) is carried out by cold condensation of the gas exiting the reactor 20, preferably by cooling the gas to a temperature comprised between 6 and 8°C.

### xi) Separating the basic mixture from algal culture

According to a preferred embodiment, the process according to the present invention comprises a further step xi), subsequent to step viii) and prior to step ix), of separation of the basic mixture 8 from algal culture, to obtain algae and a basic solution 10 from algal culture, said basic solution 10 from algal culture being fed in step ix) as an alternative to the basic mixture 8 from algal culture. In accordance with this preferred embodiment, the algae are separated and are therefore not recycled as biomass in step ii), while the basic solution 10 from algal culture is fed in step ix), to then be recycled according to one of the alternatives ix.i) and ix.ii) in place of the basic mixture 8 from algal culture.

Preferably, step xi) is carried out in a dedicated solid-liquid separator 18, preferably consisting of a dynamic separation system, configured for the separation of algae by centrifugation, microfiltration or filter pressing.

### System for carrying out the process of the invention

A preferred embodiment of a system for implementing the process according to the present invention is described below. The system in accordance with this embodiment comprises:
- a reactor 11, configured to receive a biomass input and carry out a dilution and alkalization of said biomass, and a subsequent alkaline hydrolysis reaction on a diluted and alkalized biomass;
- a drip or spray column reactor 12, in fluid communication with the reactor 11, and configured to receive as input hydrolysed biomass exiting the reactor 11 and subjecting said hydrolysed biomass to an ammonia stripping reaction by diffusing a biogas into the reactor 12;
- a column reactor 14, in fluid communication with the reactor 12, and configured to receive a deammonified biomass inputting and subjecting said deammonified biomass to a neutralization reaction, by diffusing the CO₂ from the biogas;
- a digester 15, configured to receive as input a neutralized biomass exiting the reactor 14 and subjecting said neutralized biomass to anaerobic digestion, to produce a biogas and a digestate, and feed the biogas produced in the reactor 14, said reactor 14 being, in turn, configured to feed said biogas into the reactor 12;
- a columnar or spray-type reactor 20 or Venturi scrubber, in fluid communication with the reactor 12, and configured to receive in input the biogas exiting the reactor 12, and subject said biogas to purification, to produce biomethane.
- a separator 16, consisting of a static gravity thickener or a dynamic separator, said separator 16 being configured to receive the digestate in input from the digester 15 and subject said digestate to separation, to produce a solid digestate and a clarified digestate;
- an algal culture system 17, in fluid communication with the separator 16 and with a flow diverter valve 19, said system 17 being configured to receive the clarified digestate as input, capable of feeding said algal culture, producing a basic mixture in output;
- the flow diverter valve 19, in fluid communication with the algal culture system 17 and the reactor 11, and configured to receive as input the basic mixture exiting the algal culture system, and feed said basic mixture according to one of the following alternatives:
   ∘ feeding the basic mixture into the reactor 11, if the basic mixture 8 has a pH ≥ 9.0; or
   ∘ feeding the basic mixture into the algal culture system 17, if the basic mixture has a pH < 9.0.

In accordance with a preferred embodiment, the system for the implementation of the process of the invention comprises a heat exchanger 13, positioned between the reactor 12 and the reactor 14, configured to cool the hydrolysed and deammoniated biomass exiting the reactor 12.

Preferably, the system for implementing the process according to the present invention comprises a solid-liquid separator 18, preferably consisting of a dynamic separation system, configured to separate the algae from their culture solution.

### EXAMPLES

It is noted that Example 1 describes a process according to WO2017/216720, and is reported below as a comparative example.

### Example 1:

1 kg of pollen (biomass) is prepared, having the characteristics shown below in Table 1.

**Table 1: biomass characterization.**

| | |
|---|---|
| ST (Total Solids) | 0.709 kg/kg |
| SV (Volatile Solids) | 0.589 kg/kg |
| Specific gravity | 0.800 kg/m3 |
| pH | 7.8 |

The biomass is diluted by adding source water, up to a volatile solids content of 1% of the volume of the biomass.

The diluted biomass is alkalized by the addition of a solution of sodium hydroxide at 20% by weight on the weight of the solution (w/w), up to a pH equal to 11.6.

To reach this pH value, a quantity equal to 0.95 kg of NaOH solution was added.

The diluted and alkalized biomass is subjected to hydrolysis at a temperature of 80°C, for a time equal to 90 minutes, under slight agitation, until the hydrolysed biomass is obtained.

The hydrolysed biomass is subjected to neutralization, at a temperature of 40°C, by diffusion of a gaseous mixture composed of 20% by volume of CO₂ and 80% by volume of methane, until a neutralized biomass having a pH equal to 7.0 is obtained.

The gas flow rate is adjusted so as to output a biomethane with a residual molar concentration of CO₂ of less than 2.5%.

The neutralized biomass was found to have the following characteristics shown in Table 2.

**Table 2: characteristics of neutralized biomass.**

| **Neutralized biomass** | |
|---|---|
| COD (on the filtrate and centrifuge) | 5.693 mg/L |
| pH | 7.0 |

The neutralized biomass is added with a dose of lyophilized microbial inoculum to provide the necessary startup bacterial load and subjected to anaerobic fermentation for a duration of 15 days, at a temperature of 38°C, obtaining a biogas and a digestate with the characteristics set out in Table 3.

**Table 3: Characteristics of the digestate and biogas exiting the anaerobic fermentation.**

| **Digestate** | |
|---|---|
| COD (on the filtrate and centrifuge) | 718 mg/L |
| Biochemical oxygen demand (BOD₅) | 240 mg/L |
| N-NH₃ | 2.296 mg/L |
| HCO₃⁻ (molar concentration M) | 0.08 mol/L |
| **Biogas** | |
| CH₄ | 80.5% v/v |
| CO₂ | 19.5% v/v |

### Example 2:

An embodiment of the process according to the present invention is described below.

1 kg of pollen (biomass) is prepared, having the same characteristics shown for the biomass used in Example 1.

The biomass was diluted by adding a basic solution obtained from an algal cultivation and having a pH of 11.2, to a concentration of volatile solids equal to 1% of the volume of said biomass.

Note that said algal cultivation was fed with a liquid solution having a bicarbonate concentration of 0.08 mol/L.

The diluted biomass was alkalized, by adding a 20% (w/w) sodium hydroxide (NaOH) solution, to a pH value of 11.6.

To reach this pH value, the basic solution requirement was equal to 0.09 kg of 20% NaOH solution, with a saving of 0.86 kg of the latter, corresponding to a saving of 90.5%, compared to the process reported in Example 1.

The diluted and alkalized biomass was subjected to hydrolysis at a temperature of 80°C for 90 minutes under slight agitation, obtaining a hydrolysed biomass.

Subsequently, two series-connected column reactors as described were fed respectively with the hydrolysed biomass, thus subjecting it first to stripping of ammonia at the same hydrolysis temperature (80°C), until reaching an ammoniacal nitrogen concentration of 350 mg/L, and subsequently to neutralization at a temperature of 40°C by means of diffusion of a biogas, until a neutralized biomass, having a pH of 7.0 is obtained.

The flow rate of the biogas was adjusted to output a biomethane with a residual molar concentration of carbon dioxide of less than 2.5%, in analogy to Example 1.

The biomass thus neutralized was found to have the characteristics shown below in Table 4.

**Table 4: characteristics of neutralized biomass.**

| **Neutralized biomass** | |
|---|---|
| COD (on the filtrate and centrifuge) | 5.693 mg/L |
| pH | 7.0 |

It should be noted that the COD value in the neutralized biomass is similar to that obtained in Example 1, demonstrating that the hydrolysis efficiency in terms of solubilization of the organic substance is similar even if carried out with an alkalization step involving the addition of a basic solution obtained from algal cultivation.

The neutralized biomass was added with the same dose of lyophilized microbial inoculum used in Example 1 and subjected to anaerobic fermentation, for a duration of 15 days, at a temperature of 38°C, obtaining a biogas and a digestate whose characteristics are shown below in Table 5.

**Table 5: Characteristics of the digestate and biogas exiting the anaerobic fermentation.**

| **Digestate** | |
|---|---|
| COD (on the filtrate and centrifuge) | 750 mg/L |
| BODs | 246 mg/L |
| N-NH₄ | 350 mg/L |
| HCO₃⁻ (molar concentration M) | 0.08 M |
| pH | 7.0 |
| **Biogas** | |
| CH₄ | 79.4% v/v |
| CO₂ | 20.6% v/v |

The digestate obtained from anaerobic digestion is subjected to separation by means of filtration using a filter with a porosity of 20 µm, obtaining a clarified digestate to be used as a cultivation broth, into which algae of the species Spirulina Platensis have been inserted.

The cultivation was carried out for 6 days in a photobioreactor with illumination by fluorescence phytolamps and maintained at a constant temperature of 32°C.

The trend of the pH and the concentration of bicarbonates during the feeding step of the algal cultivation is shown in Figure 4.

From this figure it is noted that the cultivation period was characterized by the constant increase in pH, reaching a value of 11.2 and, at the same time, by the constant decrease in the concentration of bicarbonates in solution, confirming the ability of algae to use bicarbonates as a source of inorganic carbon to produce a basic mixture. It should be noted that, at the end of the algal cultivation period, the concentration of residual bicarbonates is extremely low.

The basic mixture produced is essentially composed of algae in suspension in basic solution.

The algae are then separated from the basic solution by filtration using a filter with a porosity of 0.25 µm. Following the algal cultivation step, 60 g of algae (dry weight) were obtained.

The filtered basic solution, having a pH of 11.2, was recycled in the biomass dilution step, making the process continuous.

## Claims

1. Process for the continuous production of biomethane from biomasses, comprising the following steps:
i) preparing a biomass (1);
ii) diluting and alkalizing the biomass (1) by adding a basic mixture from algal culture and an alkaline solution (2), up to a pH comprised between 9.0 and 13.0;
iii) subj ecting the diluted and alkalized biomass to alkaline hydrolysis, at a temperature comprised between 55 and 90°C;
iv) subjecting the hydrolysed biomass to ammonia stripping by diffusion of a biogas, to obtain a hydrolysed and deammonified biomass;
v) neutralizing the hydrolysed and deammonified biomass, coming from step iv), by diffusing the CO₂ present in a biogas, to obtain a neutralized biomass;
vi) subjecting the neutralized biomass, coming from step v), to anaerobic digestion, to obtain a biogas (4) and a digestate (5), and recycling said biogas (4) in step v), and subsequently from step v) in step iv);
vii) subjecting the digestate (5) to a separation, to obtain a solid or semi-solid digestate (6) and a clarified digestate (7);
viii) feeding the clarified digestate (7) into a prepared algal culture (17), to obtain a basic mixture (8) from the algal culture;
ix) recycling said basic mixture (8) from algal culture according to one of the following alternatives:
ix.i) the basic mixture (8) is recycled in step ii) of dilution and alkalization of the biomass (1), if said basic mixture (8) has a pH ≥ 9.0; or
ix.ii) the basic mixture (8) is recycled upstream of step viii), if said basic mixture (8) has a pH < 9.0;
x) subjecting the biogas, coming from step vi), to purification, by removing gaseous ammonia and residual moisture, to obtain biomethane.

2. Process according to claim 1, wherein the biomass (1) is chosen from: biological sludge, algae, organic waste or by-products, organic scraps, preferably organic scraps coming from separate and undifferentiated collection, process waters, treated and untreated residual sludge, preferably coming from urban, domestic and/or industrial wastewater treatment plants, and/or treated and untreated residual sludge coming from production processes.

3. Process according to claim 1 or 2, wherein in step ii) the biomass (1) is subjected to dilution and alkalization, up to a content of volatile solids comprised between 0.5 and 10.0%, preferably comprised between 0.7 and 8.0%, preferably comprised between 0.8 and 1.5%, preferably equal to 1.0% by weight with respect to the volume of said biomass 1 (w/v).

4. Process according to any one of claims 1 to 3, wherein the alkaline hydrolysis step iii) is carried out for a time comprised between 30 and 120 minutes, preferably equal to 90 minutes.

5. Process according to any one of claims 1 to 4, comprising a step xi), subsequent to step viii) and prior to step ix), of separation of the basic mixture (8) from algal culture, to obtain algae and a basic solution (10) from algal culture, said basic solution (10) from algal culture being fed in step ix) as an alternative to the basic mixture (8) from algal culture.

6. Process according to any one of claims 1 to 5, wherein the removal of the gaseous ammonia in step x) is carried out by acid scrubbing, preferably by washing the biogas stream with a sulfuric acid solution, preferably a 20% aqueous sulfuric acid solution.

7. Process according to any one of claims 1 to 6, wherein the ammonia stripping step iv) is carried out in a drip column reactor (12), loaded with filler material, or in a spray reactor (12) or Venturi scrubber, said reactor (12) being provided with a lower accumulator with recirculation system, configured to recycle the hydrolysed biomass subjected to ammonia stripping at the top of the reactor column (12).

8. Process according to any one of claims 1 to 7, wherein the neutralization step v) is carried out in a drip column reactor (14), loaded with filler material, and saturated with a biogas, said reactor (14) being provided with a lower accumulator with recirculation system, configured to recycle the biomass to be neutralized at the top of the reactor column (14).
